# EUROPEAN PATENT SPECIFICATION

(11) **EP 3 946 121 B1**
(45) Date of publication and mention of the grant of the patent: **06.08.2025**
(21) Application number: 20779793.7
(22) Date of filing: 23.03.2020
(51) Int. Cl.: A61B 18/14, A61N 5/10, A61B 6/50, A61B 6/03, A61B 8/00, A61B 8/08, A61B 8/12, A61B 18/18, A61B 34/30, A61B 34/20, A61B 90/00, A61N 7/02, A61B 18/24, A61B 6/00, A61B 18/02

(54) **SYSTEMS FOR OPTIMIZING DELIVERY OF RADIATION TO TREAT CARDIAC ARRHYTHMIAS**
SYSTEME ZUR OPTIMIERUNG DER ABGABE VON STRAHLUNG ZUR BEHANDLUNG VON HERZARRHYTHMIEN
SYSTÈMES POUR OPTIMISER LA DISTRIBUTION DE RAYONNEMENT POUR TRAITER DES ARYTHMIES CARDIAQUES

(30) Priority: 24.03.2019 US 201916362673
(43) Date of publication of application: 09.02.2022
(73) Proprietor: Ablation Innovations, LLC, Brentwood, TN 37027 (US)
(72) Inventor: KAISER, Daniel Walter, Nashville, TN 37210 (US); ALLEY, Christopher B., Brentwood, TN 37027 (US)
(74) Representative: Boult Wade Tennant LLP
(86) International application number: PCT/US2020/024308
(87) International publication number: WO 2020/198165

(56) References cited:
- WO-A1-2017/078757
- WO-A2-2008/086434
- US-A1- 2005 261 672
- US-A1- 2005 261 672
- US-A1- 2008 039 746
- US-A1- 2008 039 746
- US-A1- 2009 171 337
- US-A1- 2014 277 219
- FRANCESCO SBRANA ET AL: "Anesthetic management in atrial fibrillation ablation procedure: Adding non-invasive ventilation to deep sedation", INDIAN PACING AND ELECTROPHYSIOLOGY JOURNAL, vol. 15, no. 2, 1 March 2015 (2015-03-01), IN, pages 96 - 102, XP055743541, ISSN: 0972-6292, DOI: 10.1016/j.ipej.2015.07.003
- SBRANA F ET AL.: "Anesthetic management in atrial fibrillation ablation procedure: Adding non-invasive ventilation to deep sedation", INDIAN PACING ELECTROPHYSIOL J., vol. 15, no. 2, 29 July 2015 (2015-07-29), pages 96 - 102, XP055743541

## Description

### FIELD OF THE INVENTION

The present invention relates to systems for optimizing radiotherapy treatment of cardiac arrhythmias.

### BACKGROUND

Atrial fibrillation is the most common sustained cardiac arrhythmia seen in clinical practice. It causes significant morbidity in both quality of life and risk of thromboembolic disease. Atrial fibrillation increases the risk of stroke four fold. The cornerstone of ablation procedures is pulmonary vein isolation (PVI). In some cases, electrical isolation of the left atrial appendage or posterior wall of the left atrium improve the success of ablation procedures.

Isolating the left atrial appendage (LAA) is associated with a reduction in atrial fibrillation recurrence. However, isolating the left atrial appendage reduces flow velocity, and increases the risk of thromboembolic events even in patients who maintain normal sinus rhythm. Therefore, there is a need to isolate the left atrial appendage without increasing the risk of stroke. Given the reduced blood flow velocity after left atrial appendage electrical isolation, the left atrial appendage is typically closed mechanically. Attempts to electrically isolate the left atrial appendage with catheter ablation have demonstrated a high incidence of electrical reconnection. Therefore, while closure devices are typically recommended after electrical isolation of the left atrial appendage, these closure devices typically prevent attempts to electrically re-isolate the left atrial appendage with catheter ablation.

Atrio-esophageal fistula (AE fistula) is the most feared complication of catheter ablation procedure of atrial fibrillation. Although a rare phenomenon, an AE fistula has a high mortality. The posterior wall of the left atrium is adjacent to the anterior wall of the esophagus. Ablation of the posterior left atrial wall during ablation procedures can also damage the esophageal tissue, which may result in the formation of an AE fistula. During ablation of pulmonary veins or the posterior wall of the left atrium, improved methods to minimize damage to the esophagus is needed.

US2005/261672 discloses methods and apparatus for selective denervation of conduction pathways in the heart for the treatment of dysrhythmias, including one or more ablation or electroporation catheters having electrodes for stimulating, targeting, and ablating fat pad tissue and other cardiac tissue to selectively denervate heart tissue.

WO2008/086434 discloses a method and system for radiosurgical treatment of moving tissues of the heart, including acquiring at least one volume of the tissue and acquiring at least one ultrasound data set, image or volume of the tissue using an ultrasound transducer disposed at a position. A similarity measure is computed between the ultrasound image or volume and the acquired volume or a simulated ultrasound data set, image or volume. A robot is configured in response to the similarity measure and the position of the transducer, and a radiation beam is fired from the configured robot.

Radiation can be used to ablate arrhythmia prone cardiac tissue. In order to determine the location of the target, fiducial leads are often placed within the body. However, placement of fiducial leads can be dangerous and uncomfortable to patients. Systems and methods to help identify target structures without placing leads into the heart would be advantageous.

Furthermore, it is difficult to keep the radiation secluded from other healthy tissue that does not need radiation therapy. Improvements are needed to allow radiation only to contact desired areas of tissue that need radiation treatment. Therefore, novel systems and methods are needed to reduce damage to the esophagus during radiation treatment to the left atrium.

In addition, tracking motion of target structures during an ablation procedure may improve ablation success and limit damage to healthy tissue. Furthermore, while x-ray therapy has been most commonly used to performed ablation procedures, charged particles can also be used. Charged particles have the advantage of limiting radiation exposure to surrounding tissue. In addition, charged particles can be influenced by magnetic fields.

Furthermore, certain areas of the heart that need to be ablated for an optimal ablation procedure are in close proximity to the esophagus. However, the esophagus is very sensitive to radiation therapy, limiting radiation to certain areas. Therefore, combining different ablation technologies with radiation therapy can be used to optimize the ablation procedure. For example, radiation therapy can be utilized to perform a large portion of the ablation procedure, particularly when there is no sensitive tissue in the treatment area. However, when there is sensitive tissue near the ablation targets, alterative ablation energies can be delivered to complete the ablation procedure. Some of these ablation energies can be delivered through the esophagus without critical damage to the esophagus.

### SUMMARY

The present invention is directed to systems to facilitate ablation of myocardial tissue to treat arrhythmias. Specifically, the present application relates to systems to improve radiation therapy to ablate cardiac tissue to treat cardiac arrhythmias.

According to the present invention there is disclosed an ablation system to treat cardiac dysrhythmias of a heart within a patient's body as claimed in the appended claims. Methods described here below are not claimed but are helpful for the understanding of the invention.

A trans-esophageal echocardiogram ("TEE") probe is described that may be placed into an esophagus of a patient to map out targets for radiotherapy. The TEE probe may function as a fiducial marker to track cardiac motioa In addition, real-time monitoring of cardiac structures by ultrasound may be used to focus radiotherapy. By combining prior computed tomography (CT) images with ultrasound images, targets may be tracked. By tracking movement of targets, such as the pulmonary veins, during the cardiac cycle and with effects of respiration, greater radiation may be delivered to targets while minimizing radiation exposure to tissue that is not targeted.

By enabling the elongated device within the esophagus to both image tissue and move the esophagus, improved visualization for improved monitoring is enabled. In addition, moving the esophagus away from the heart will enable radiation therapy to be delivered to critical tissue while minimizing damage to the esophagus. In another embodiment, the TEE probe-like device may be designed to create a magnetic field. The magnetic field may be used to optimize radiotherapy.

For example, the distance between the posterior left atrium and esophagus may be a few millimeters. The magnetic field may be controlled from within the esophagus using the probe to minimize radiotherapy to the esophagus while maximizing radiotherapy to target structures, such as the posterior wall. The esophagus probe may adjust the magnetic field to prevent radiation from reaching the esophagus. By creating the magnetic field from directly within the esophagus, the charged radiation may be controlled to target the pulmonary veins and posterior wall of the left atrium while minimizing radiation damage to the esophagus. This may help prevent atrio-esophageal fistulas from forming.

In other embodiments, the transesophageal device may change the temperature, orientation, or physically move the esophagus relative to the patient's heart to minimize damage to the esophagus. The transesophageal device may move during a radiotherapy treatment to optimize radiotherapy to critical tissue. In another embodiment, negative pressure may be delivered within the esophagus to bring the esophageal tissue to contact the transesophageal device. In some embodiments, the device may include one or more balloons, which may be inflated within the esophagus to maintain a negative pressure around the transesophageal device. By creating a negative pressure within the esophagus, the location, orientation, and/or position of the esophagus may be better monitored and controlled.

In another embodiment, the device within the esophagus may be mechanically transformed from an initial delivery shape into an expanded or other shape, such as a wedge or pyramid, to optimize radiation to target tissue while minimizing radiation to non-target tissue. By moving the esophageal device towards the left atrium or increasing the pressure inside of the esophagus higher than the pressure inside the left atrium, the posterior wall of the left atrium may be positioned to maximize radiation exposure to targets while minimizing radiation damage to non-targets. In another embodiment, a compliant or noncompliant balloon may be inflated within the esophagus to prevent the device from moving within the esophagus.

In another embodiment, the transesophageal device may be connected to a motor and/or a robot. The robot or motor may be activated to move the device within the esophagus. Therefore, while delivering radiation therapy to key aspects of the tissue, the device may then be used to move the esophagus to a different location. By moving the esophagus within the thoracic cavity and/or relative to the patient's heart, additional radiation therapy may be delivered to desired regions or aspects of the tissue, e.g., to treat a dysrhythmia, while minimizing the radiation to the esophagus and/or other critical structures. In one embodiment, by having a robot or motor move the device, radiation may be delivered without requiring a person within the treatment room.

In some circumstances, the esophagus cannot be adequately moved to deliver radiation therapy safely. In these and other circumstances, delivering energy through the esophagus may improve an ablation procedure. There are a variety of energy sources that may be delivered through the esophagus to target tissue while minimizing injury to the esophagus. For example, electroporation energy, focused high intensity ultrasound energy, radiation energy, radio frequency energy, microwave energy, cryothermal energy, and laser energy may be delivered through the esophagus to damage critical tissue to improve the ablation procedure. Electroporation ablation utilizes pulsed, high voltage energy to induce electroporation of cells followed by cell death. This ablation technology is tissue specific and is non-thermal. Prior studies demonstrate that electroporation delivered directly to esophageal tissue results in necrosis of the muscular layers while leaving the endothelial layers unaffected. Thus, this ablation energy may be less likely to cause atrio-esophageal fistulas compared to other ablation energy technologies.

In another embodiment, the transesophageal device may be used to twist the esophagus. For example, the device may include a ballooti such that the esophagus may be advanced forward or withdrawn posteriorly or laterally to control positioning of the esophagus. In another embodiment, the device may include spaced-apart balloons, and the balloons may be inflated at distal and proximal locations. Then, negative pressure may be applied to the isolated region of the esophagus between the balloons to cause the esophagus to collapse around the elongated device within the esophagus. In another embodiment, an endotracheal tube including multiple lumens may be introduced into the patient's lung such that one or both of the lungs may be deflated. By collapsing a lung, the esophagus may be moved to a better location. Furthermore, collapsing a lung may enable better imaging by a transducer carried on the device and positioned within the esophagus.

**In** another embodiment, a magnetic field may be created by the elongated device within the esophagus. By combining a magnetic field from within the esophagus with positioning of the posterior wall of the esophagus, treatments may be designed to minimizing damage to the esophagus. For example, in one embodiment charged radiation may be delivered at an angle to the posterior left atrium that has been positioned and combined with the magnetic field to maximize exposure to one or more target tissue regions while minimizing damage to non-target structures. In some embodiments, radiation may be allowed to be delivered to the blood pool where the risks of radiation are less likely to build over time. Charged radiation includes proton therapy and/or carbon ion therapy.

The device may also cool the esophageal temperature to minimize the effects of radiation on the tissue. The esophageal device may also function as a fiducial marker to help guide radiation therapy.

In another embodiment, the transesophageal device may create a fluctuating magnetic field. For example, the magnetic field may be designed to"bend," stop, or maximize radiotherapy to certain targets. In other embodiments, the magnetic field is designed to force radiotherapy to stop prior to contacting esophageal tissue. Therefore, while radiation may be directed to the target region(s) from a plurality of angles, adjusting the magnetic field around the body or within the esophagus may further optimize radiation include the esophagus, coronary arteries, nerve tissue, and valvular tissue.

The transesophageal device may be placed within the esophagus and ultrasound images may be obtained. Next, a CT scan may be performed with the esophageal device in place. Contrast may or may not be used during the CT scan. Then, images from the CT scan may be combined with the images from the ultrasound to better identify tissue within the subject. By combining fiducial markers that can be tracked by both ultrasound monitoring and radiation (including x-rays), treatment may be optimized.

The radiation ablation procedure may be performed after a device has been placed in the left atrial appendage. The device placed in the left atrial appendage may be or similar to a Watchman device or the Amulet. Therefore, in one embodiment, radiation therapy may be delivered to the heart, and the device in the left atrial appendage may be used as a fiducial marker to guide radiotherapy. This may be used in addition to the elongated device placed within the esophagus.

In accordance with an exemplary embodiment, an ablation system is provided to treat cardiac dysrhythmias of a heart within a patient's body that includes an elongate member having a proximal end and a distal end sized to be placed within an esophagus within the patient's body, the elongate member comprising an energy-delivering portion on the distal end, wherein the energy-delivering portion delivers energy through the esophagus to ablate tissue in order to treat cardiac dysrhythmias.

A method is described for treating cardiac dysrhythmias in a heart within a patient's body that includes introducing a distal end of an elongated member into an esophagus within the patient's body; and delivering energy from one or more elements on the distal end through the esophagus to ablate tissue in order to treat cardiac dysrhythmias.

An ablation system is provided to treat cardiac dysrhythmias of a heart within a patient's body that includes a machine configured to deliver radiation energy into the patient's body; a device designed to deliver
electroporation energy from within the esophagus; and a controller coupled to the machine and the device such that the two energies are delivered during the same procedure to treat cardiac dysrhythmias.

A method is provided for treating cardiac dysrhythmias in a heart within a patient's body that includes providing a radiation delivery machine adjacent the patient's body; introducing a distal end of an elongated member into an esophagus within the patient's body; and selectively delivering electroporation energy from one or more elements on the distal end from within the esophagus and activating the machine to deliver radiation energy into the patient's body such that both electroporation energy and radiation energy are delivered during the same procedure to treat cardiac dysrhythmias.

Other aspects and features of the present invention will become apparent from consideration of the following description taken in conjunction with the accompanying drawings.

### BRIEF DESCRIPTION OF THE DRAWINGS

The invention is best understood from the following detailed description when read in conjunction with the accompanying drawings. It will be appreciated that the exemplary apparatus shown in the drawings are not necessarily drawn to scale, with emphasis instead being placed on illustrating the various aspects and features of the illustrated embodiments.
FIG. 1 is a cross-sectional view of a patient's body showing an exemplary embodiment of a transesophageal device positioned within the patient's esophagus.
FIG. 2 is a cross-sectional view of a patient's body showing another exemplary embodiment of a transesophageal device positioned within the patient's esophagus.
FIG. 3 is a block diaphragm showing exemplary components that may be included in one embodiment of a system.
FIG. 4 is a flow diaphragm showing an exemplary method for using one embodiment of a system to perform an ablation procedure.
FIG. 5 shows an exemplary embodiment of a system combining radiation therapy within a patient' s heart with ultrasound guidance from within the patient' s esophagus.
FIG. 5 A is a detail showing a transesophageal device positioned within the esophagus adjacent the patient's heart.
FIG. 6 is a cross-sectional detail of a patient's body showing an exemplary embodiment of a transesophageal device placed within the patient's esophagus.

### DETAILED DESCRIPTION OF THE EXEMPLARY EMBODIMENTS

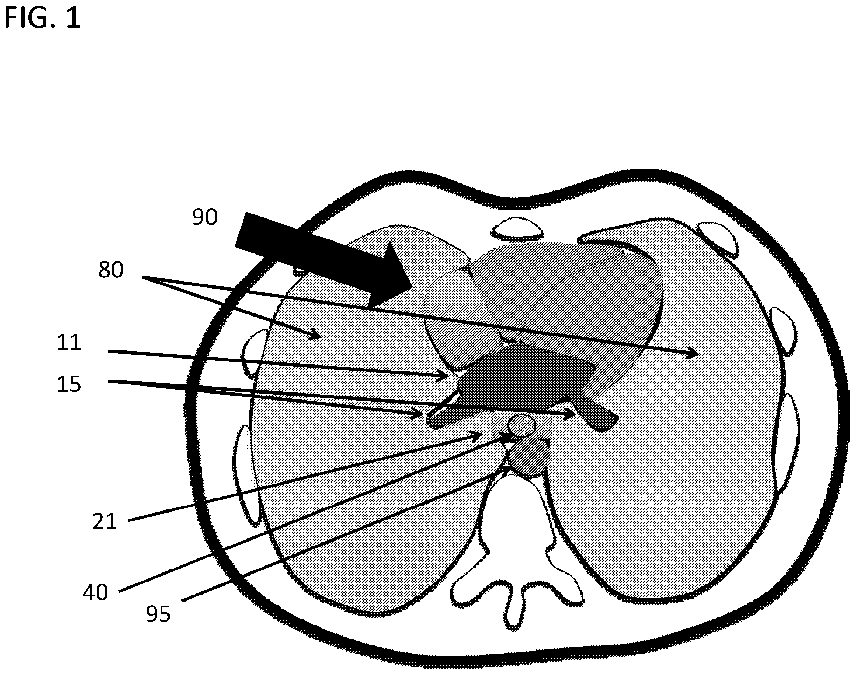

Turning to the drawings, FIG. 1 shows an exemplary embodiment of a system to facilitate delivery of radiation to a patient's heart 90 while minimizing radiation to critical tissues. FIG. 1 shows a cross-section of the patient's chest that demonstrates the heart 90 and lungs 80. The left atrium 11 of the heart 90 is the posterior aspect of the heart 90 just anterior to the esophagus 21. Blood enters the left atrium 11 through the pulmonary veins 15.

In accordance with an exemplary embodiment, the system includes a transesophageal device 40, which is shown being positioned within the esophagus 21, and a radiation delivery device (not shown). As described elsewhere herein, the radiation delivery device is a machine external to the patient that may be positioned and oriented towards the patient's body, e.g., operated using a robot or other controller to deliver radiation energy into the patient's body from one or more locations, and/or may be introduced internally, e.g., into the patient's heart 90 as part of the device 40 or as a separate device. Generally, the transesophageal device 40 includes a proximal end (not shown), e.g., including a handle and/or one or more actuators (also not shown), and a distal end sized for introduction into the esophagus 21 that carries one or more sensors, markers, and/or actuatable components, as described elsewhere herein.

For example, the device 40 may include one or more sensors and/or imaging elements having imaging capabilities (e.g., ultrasound to visualize key structures). The distal end of the device 40 may also include one or more markers, e.g., to function as a fiducial for x-rays or other imaging modalities, e.g., to be linked to a three-dimensional mapping/imaging system, such as ultrasound or impedance-based mapping systems. The device 40 may also have a magnet or one or more electromagnetic elements configured to help facilitate mapping, imaging, or guidance of radiation.

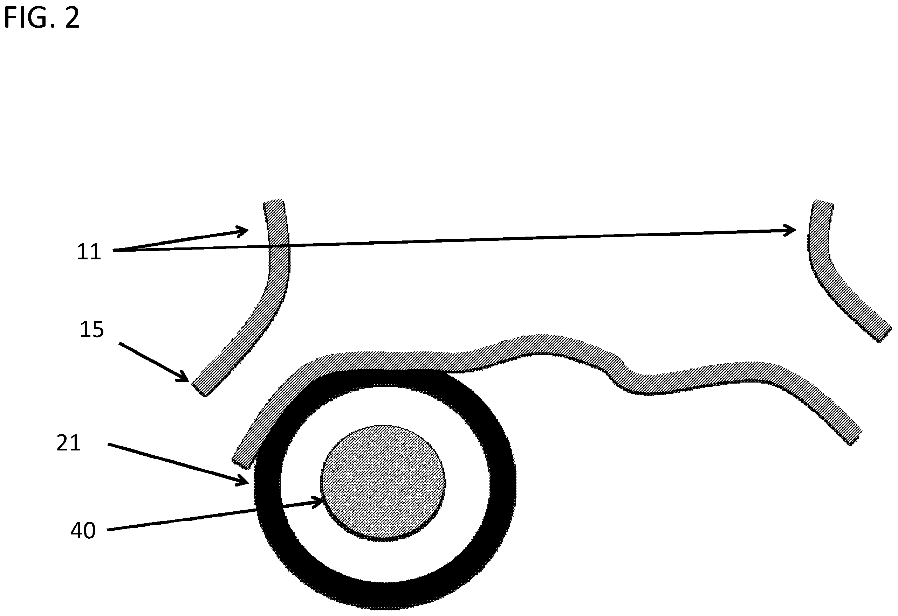

FIG. 2 shows another exemplary embodiment of a system for delivering radiation to the heart using a transesophageal device 40. As may be observed from this detail, the esophagus 21 is generally located in close proximity to the pulmonary veins 15. In this embodiment, the device 40 includes one or more balloons carried on the distal end (not shown) to help position the device 40 within the esophagus 21 and/or manipulate the esophagus 21 in a desired manner. The balloon may be filled with material that optimizes the delivery of ultrasound images. By combining images with a stable position, the walls of the esophagus 21 may be combined with a mapping system to minimize radiation to the esophagus 21.

In another embodiment, the distal end of the device 40 (within the esophagus 21) may be actuated to change shape, e.g., to move the esophagus 21 away from the left atrium 11, position the left atrium 11 and pulmonary veins 15, and/or otherwise manipulate the esophagus 21, e.g., such that delivery of radiation is tangential to the esophagus 21. Optionally, the distal end of the device 40 may include one or more elements to generate magnetic forces to further optimize delivery of radiation.

In another embodiment, the distal end of the transesophageal device 40 may include a positively or negatively charged electrode, or other elements to create an electric field, which may be used to help guide, control, or direct radiation from the radiation delivery device. In another embodiment, one or more charged electrodes may be provided on the distal end to function as a sink to attract radiation or expel radiation away from the device 40 to protect the esophagus 21.

In another embodiment, the system may include one or more patches that may be placed on the exterior chest of the patient (not shown). The patches and the esophageal device 40 may function as a fiducial for x-ray therapy. In addition or alternatively, the patches and esophageal device 40 may help to create a map using external ultrasound and/or impedance mapping. By combining the fiducials from x-rays with the created map, targets for radiation may be monitored in real-time. Therefore, during the cardiac cycle, patient movement, or patient breathing, real-time monitoring of structures within the patient may facilitate delivery of radiation by the radiation delivery device.

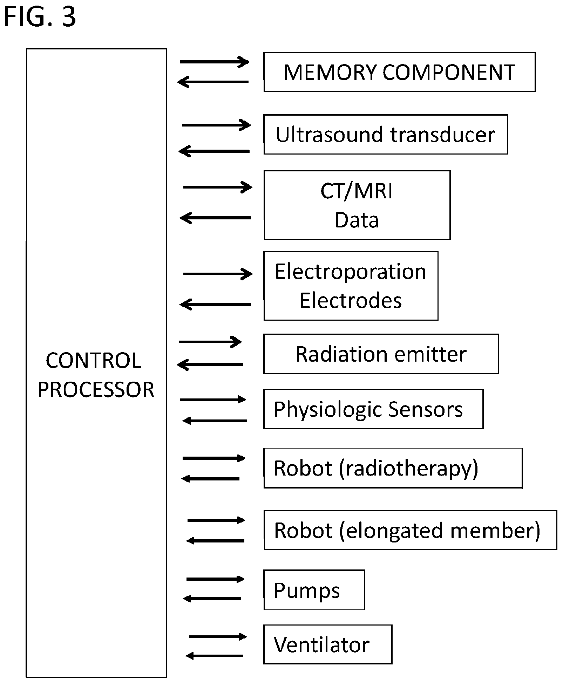

Turning to FIG. 3, a block diaphragm shows exemplary components that may be included in one embodiment of a controller of the system, e.g., coupled to and/or communicating with a transesophageal device and/or other components of the system (not shown). The controller includes a control processor that communicates with and/or controls other components of the system in order to map and track the heart as well as direct ablation energy to perform an ablation procedure. For example, as shown, the control processor is connected to a memory component to store and access data.

In addition, the control processor may be connected to an ultrasound transducer, e.g., carried on the distal end of the transesophageal device, which may be positioned within the esophagus. The controller may receive signals from the transducer to track cardiac tissue during the cardiac cycle, as well as based on patient movement (including breathing), e.g., while the distal end and transducer are moved within the esophagus, e.g., by manipulating the transesophageal device from outside the patient's body.

In addition or alternatively, sometimes it may be desirably to manipulate the distal end of the transesophageal device to move and/or distort the patient's anatomy. For example, the distal end may include one or more expandable members, e.g., balloons, mechanically expandable structures, and the like (not shown), e.g., to increase an outer profile and/or shape of the distal end. In one embodiment, the external aspects of the device may be fixed, while internal components may be actuatable, e.g., using one or more actuators on the proximal end, to move without effecting the patient's anatomy in order to optimize delivery of ablation energy without requiring repeat scans to identify the anatomy.

In addition, as shown in FIG. 3, the control processor may also be connected to CT or MRI imaging scan files, e.g., within the controller and/or accessible from an external source (not shown). These scans may be performed prior to the ablation procedure or may be performed in real-time during the ablation procedure. The proceduralist may use data from the files and/or real-time data to identify one or more target tissue locations or structures for ablation, e.g., based on rendering of the images on a display coupled to the controller (also not shown).

The control processor may also be coupled to one or more electroporation electrodes, e.g., carried on an external radiation delivery device that may be positioned externally on or around the patient's body. In other embodiments, these electrodes may be configured to delivery other forms of ablation energy used to ablate cardiac tissue, such as focused high intensity ultrasound energy, radiation energy, radiofrequency energy, microwave energy, cryothermal energy, laser energy, and the like.

According to the invention, the control processor is coupled to a radiation emitter included in an external radiation delivery device. This may be a linear accelerator or other source of radiation. The radiation emitter may be controlled by a robot (not shown) communicating with or contained within the controller to direct radiotherapy to the target structures within the patient's body as determined by the proceduralist(s).

In addition, the control processor is also coupled to one or more physiologic sensors, e.g., placed externally to the patient's body. These sensors may include surface electrodes to monitor the cardiac cycle, respiratory sensors to monitor the breathing cycle, movement sensors, and other physiologic sensors. The control processor may combine physiologic sensor data from these sensor(s) with imaging data to track and model how various structures within the patient's body move during the procedure. By modeling how the structures in the patient's body move during the procedure, energy may be delivered to the target locations while minimizing error.

As shown in FIG. 3. the control processor may also be coupled to at least one robot. For example, the system may include at least one robot programmed to control radiation beams from the radiation delivery device. This robot may be similar to other radiation therapy systems that utilize a robot to control the radiation source. In another embodiment, a second robot may also be coupled to the control processor. This robot may control various aspects of the transesophageal device placed within the esophagus. For example, the second robot may control the location, orientation. and/or rotation of electroporation electrodes and/or ultrasound transducer(s) carried on the distal end of the device within the patient's esophagus. In addition, the second robot may be configured to manipulate the distal end of the device to move the esophagus within the chest, e.g., posteriorly and/or laterally within the chest cavity, to move the esophagus to different locations in relation to the heart 90.

For example, with additional reference to FIG. 1, the second robot may manipulate the device 40 to move the esophagus 21 to the right within the patient's body while radiation therapy is delivered to the left pulmonary veins 15. Then, the robot may manipulate the device 40 to move the esophagus 21 to the left within the patient's body enabling radiation therapy to be delivered to the right pulmonary veins 15 while minimizing radiation therapy to the esophagus 21.

Furthermore, with reference again to FIG. 3, in another embodiment the control processor may be coupled to one or more pumps. These pumps may be used to control fluid flow into and out of a chamber or expandable element carried on the distal end of the transesophageal device. In an exemplary embodiment, the expandable element may include one or more compliant or non-compliant balloons carried on the distal end of the device. The expandable element(s) may be used to move the esophagus within the patient's body, similar to the robot described above. In addition or alternatively, the expandable element(s) may be selectively expanded and/or contracted to provide better spatial control of the esophagus and/or to provide enhanced contact with various imaging transducers and electrodes carried on the distal end of the device and positioned within the patient's body. For example, the esophagus may be moved spatially within the chest cavity by inflating different chambers provided on or within the distal end of the transesophageal device. In another embodiment, the pumps are used to control the esophagus, with or without the help of a motorized robot. The pumps may selectively pump air, water, or other fluid into and/or out of the chambers. Optionally, the fluid may also be substantially radio-opaque. e.g. to act as a fiducial marker during the ablation procedure.

The control processor may also be coupled to a ventilator. This may be useful because respirations not only move markers or targets that may be provided on the patient's skin or introduced within the heart or elsewhere in the chest cavity, but lung volumes/pressures influence imaging windows and the orientation of structures within the chest. Therefore, the optimal time to deliver radiation therapy may be with the lungs collapsed or fully expanded In some embodiments, the ventilator is designed to control single lung respiration. In one embodiment, this enables one lung to be collapsed while the other lung is ventilated. By controlling the ventilation, ultrasound imaging, target modeling, and radiation delivery may be optimized by the controller. In some circumstances, the patient is sedated and paralyzed so that the ventilator has complete control over respirations.

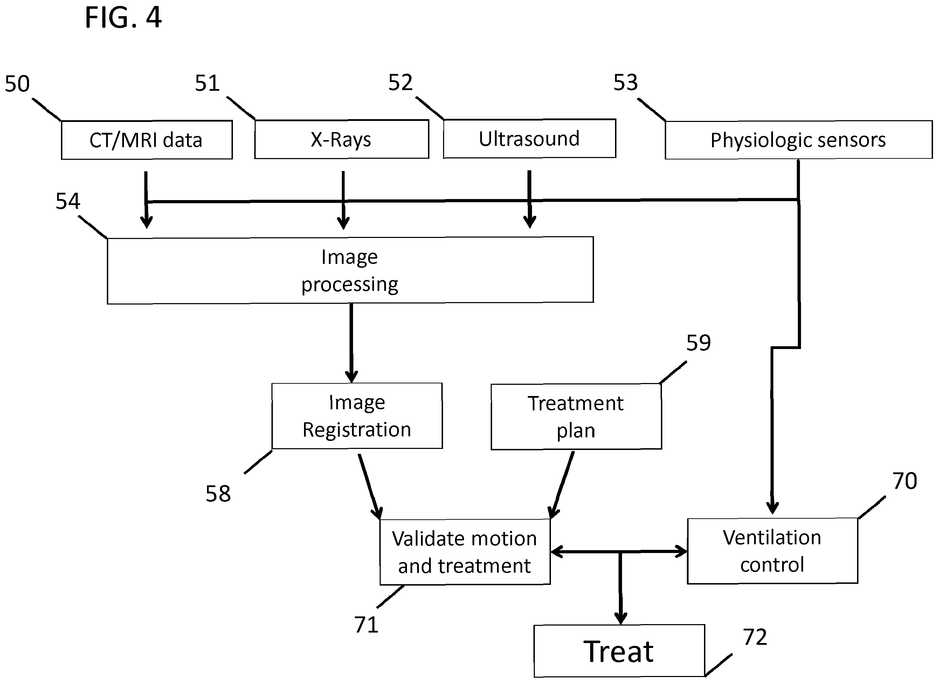

Turning to FIG. 4, an exemplary flow diaphragm is shown of one method for performing a medical procedure, e.g., an ablation procedure, within a patient's body using a system, such as those described elsewhere herein. The system obtains images from

CT/MRI data 50, X-rays 51, and ultrasound imaging 52. These images may be taken prior to the ablation procedure or performed in real-time or immediately before the ablation procedure. The system uses these images to identify anatomical structures within the body and registers the images into a coherent map structure. An elongated member, e.g., any of the transesophageal devices described herein, may be positioned in the patient's esophagus, which may serve as a fiducial marker to orient the CT/X-ray images. In addition, the ultrasound images may be registered with other imaging to orient the maps together. These images are recorded with along with data from physiological sensors 53. These sensors obtain data from one or more of electrocardiogram, respiratory, impedance, and movement sensors in order to track and coordinate targets in 3D space. In some examples, ventilation control 70 is also utilized to control the ventilation/respiration of the patient.

Image processing at step 54 may include various filtering modalities, various mapping techniques, gamma correction, image enhancement, spectral processing, and/or the like. The image data from the various modalities are combined with the data from the physiologic sensors and undergo image processing 54. Once the image processing 54 has been combined with physiologic sensors data 53, these data undergo image registration 58. Image registration 58 generally involves spatially transforming the various images to align with the target image. In some cases, the target and surrounding tissue are determined a priori utilizing an imaging modality. This imaging is used to create a treatment plan 59.

The treatment plan 59 may include identifying the target volume or location, and the desired dose to the target is prescribed. Sensitive tissue structures (or critical structures) in the vicinity of the target are also delineated and may be assigned a maximum dose that can be deposited to these structures. A computer program, e.g., within the controller of the system or an external device communicating with the controller, then receives the various targets and critical structures, the prescribed doses, the geometric configuration of the radiation delivery system, and the ventilation control 70 in order to compute the position and orientation of beams from the radiation delivery device paired with the ventilation in order to determine and optimize the treatment plan 59. The computer program also determines the radiation dose received by all tissue. This data can be reviewed by the proceduralist(s) to verify the radiation to the target and critical structures. The treatment plan 59, image registration 58, and ventilation control 70 are all then combined to validate motion and treatment 71.

Once the treatment plan has been validated, ablation therapy may be delivered to treat the patient at step 72. Treating the patient may include delivering radiation therapy and/or other ablation technologies from within the esophagus, e.g., using one or more treatment elements carried by the distal end of the transesophageal device. The treatment may also include using one or more robots, e.g., to control patient ventilation as well as the ablation technologies, e.g., one or both external to the patient and from within the esophagus. The proceduralist(s) must also verify the plan for ventilation control 70 is appropriate for the patient receiving therapy. This may include collapsing a lung, prolonged breath holds, hyperventilation, and the like in order to optimize the ablation procedure. However, certain maneuvers or duration may not be safe for all patients.

Therefore, the proceduralist(s) must verify all aspects of the plan before proceeding with the treatment.

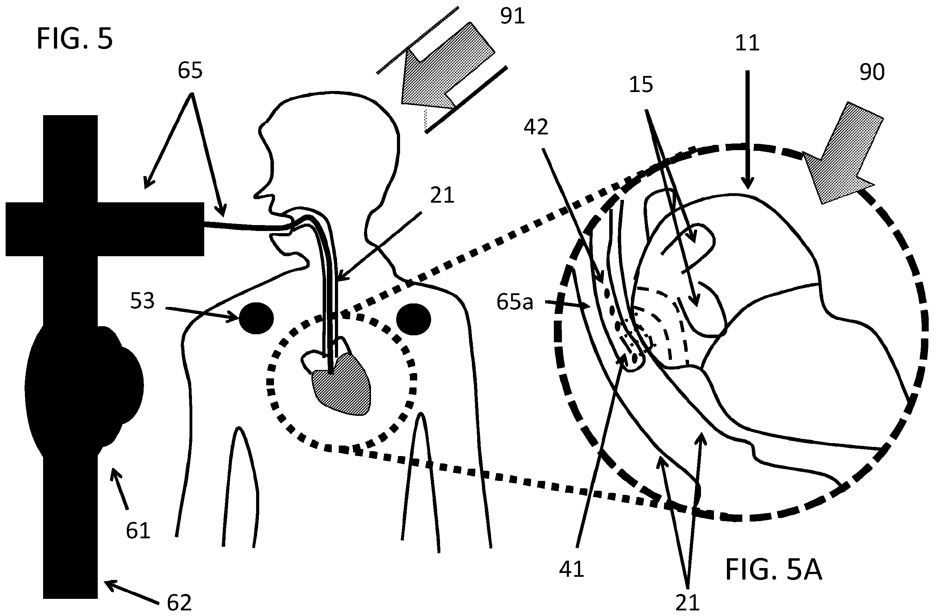

Turning to FIG. 5, an exemplary embodiment of a system is shown for performing a medical procedure combining radiation therapy with ultrasound guidance from within the esophagus. In this figure, a patient 91 is shown with a heart 90 prepared to undergo an ablation procedure. The patient 91 has an elongated member 65, e.g., similar to any of the transesophageal devices described elsewhere herein, designed to be placed within the esophagus 21 of the patient 91. The elongated member 65 may also have motorized controller and/or a robot that may be actuated to move the esophagus 21 to an optimal orientation relative to the heart 90, e.g., to multiple sequential locations as ablation energy is delivered. The system also includes a radiotherapy device 61 external to the patient 91 that is controlled by a robot or other controller (not shown) to control the delivery and angles of radiation. The patient 91 also has one or more physiologic sensors 53, e.g., placed on the patient's skin and/or other locations, which the controller may use to record one or more of movement, breathing, impedance, and electrocardiograms.

As shown in the detail of FIG. 5 A, the patient's 90 is located in front of the esophagus 21. The distal end 65a of the elongated member 65 may be advanced into the esophagus 21, e.g., to position an ultrasound transducer 41 and/or other elements carried thereon within the esophagus 21. The controller may receive data from this transducer 41 to track target structures within the heart 90, e.g., including the left atrium 11 and pulmonary veins 15.

In addition, the elongated member 65 carries one or more energy-delivering portions, e.g. electrodes 42 on the distal end 65a. These electrodes 42 may be used to deliver electroporation ablation to the target structures. These electrodes 42 may be configured in various shapes and sizes, including elongated electrodes used in various other electroporation ablation procedures. Furthermore, these or other electrodes may be provided on the distal end 65a to monitor physiological signals to guide the ablation procedure. In some embodiments, the system also includes ventilation control 70 (not shown), similar to other embodiments described herein. In addition, the patient 91 will be dosely monitored during the ablation procedure, including pulse oximetry, heart rate, and blood pressure (not shown.

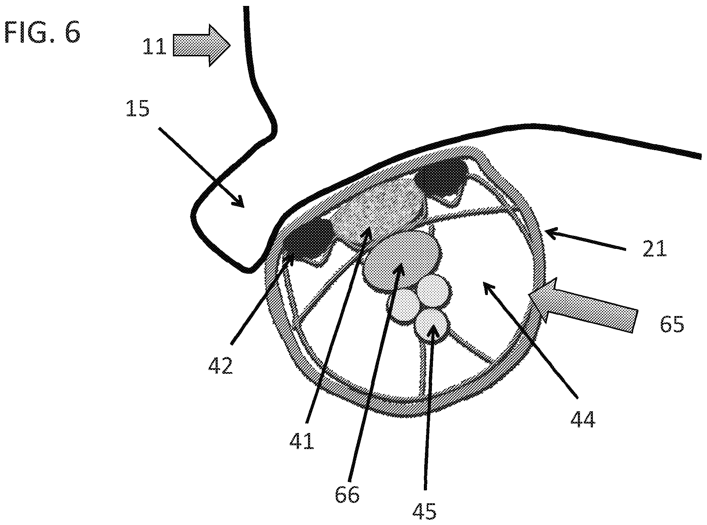

Turning to FIG. 6, another exemplary embodiment of a system is shown that includes an elongated member 65 including a proximal end (not shown) for manipulating and/or actuating the elongate member 65, and a distal end configured to be introduced within a patient's esophagus 21, generally similar to other embodiments herein. In this figure, the elongated member 65 may positioned such that the distal end is located within the esophagus 21, e.g., behind the left atrium 11, and used to visualize and track target structures. In addition, a controller of the system can track and/or ablate the pulmonary veins 15 or posterior wall of the left atrium 11. The elongated member 65 carries one or more electrodes 42 on the distal end configured for recording electrical signals as well as delivering electroporation ablation energy. Optionally, an ultrasound transducer 41 may also be carried on the distal end, similar to other embodiments herein.

Optionally, the elongated member 65 may also include a motorized control portion 66, e.g., on the distal end, capable of moving the esophagus 21 in space and/or rotating the esophagus 21 when actuated. Alternatively, the motorized control portion 66 may move components inside of the elongated member 65 without affecting the esophagus 21.

In some embodiments, the elongated member 65 may include one or more chambers 44 carried on or within the distal end, e.g., defined by balloons or other enclosed structures, e.g., formed from compliant, non-compliant, or substantially non-compliant material. Gas, water, or other fluid may be introduced into and/or removed from the chambers 44 to selectively expand and contract the structures. For example, by inflating one or more of the chambers 44, the distal end of the elongated member 65 may have better contact with the esophagus 21 or have better control in moving the esophagus 21.

Furthermore, in some embodiments, the elongated member 65 may include one or more lumens 45 extending between the proximal and distal ends of the elongated member 65. These lumens 45 may have access to the gastric fluid or provide the ability to inflate or deflate one or more of the chambers 44. In some embodiments, inflation of one chamber 44 will move the esophagus 21 in one direction while inflation of another chamber 44 will move the esophagus in the opposite directioa This movement may be performed with or without the support of the motorized control portion 66. In addition, the esophagus 21 may be selectively manipulated using the various described mechanisms in combination with the ventilation control 70, since respiration and intrathoracic pressure affects the ability to move the esophagus in relation to the heart 90. In addition, in some embodiments, the lumens 45 may contain electromagnetic material, which may create a magnetic field capable of directing charged radiation away from critical tissue and towards target structures.

According to the invention, the elongated member 65 carries one or more electroporation electrodes 42 on the distal end. In addition, the elongated member 65 may have a variety of energy-delivering portions, including focused high intensity ultrasound energy, radiation energy, radiofrequency energy, microwave energy, cryothermal energy, and laser energy. Electroporation ablation is tissue specific and can safely ablate nearby tissue without critical damage to the esophagus 21. In some cases, a plurality of electrodes 42 are positioned spaced apart along the distal end of the elongated member 65. However, in other embodiments, at least one electrode 42 may be provided to deliver the electroporation ablation. These electrodes 42 may then be coupled to an electrical pulse generator, e.g., via the controller, to deliver ablation energy. By combining some form of ablation from the esophagus 21 with an external radiotherapy device 61, a complete ablation procedure may be performed without substantial risk of injury to critical tissues, including the esophagus 21.

The ablation procedure may be performed with the patient intubated, sedated, and/or on a ventilator and be used to treat a variety of cardiac dysrhythmias, including atrial fibrillation. The ablation energy from the elongated member 65 may be used to ablate cardiac tissue, pulmonary vein tissue, and/or ganglionic plexi, as prescribed by the proceduralist(s). Controlling ventilation is paramount for the ablation procedure. Lung volumes influence the relationship between the left atrium 11 and the esophagus 21. The volumes influence the angles of radiation ablation as well as the ability of ultrasound transducer 41 to map and tract tissue. Therefore, in some embodiments, a processor (not shown) controls the ventilator (not shown) in order to optimize the ablation procedure. Furthermore, in some situations the esophagus 21 may need to be moved. Therefore, in some embodiments, a motorized control portion and/or robot 66 may be coupled to the distal end of the elongated member 65, which may manipulate the distal end to position or control the elongated member 65. The robot 66 may be used to move the esophagus 21 relative to the heart 90.

The external radiation source 61 (shown in FIG. 5) is typically a linear accelerator capable of generating an X-ray beam. The external radiation source 61 typically has a single source of radiation that is moved around the patient by a machine or robot arm 62. In addition, another robot (not shown) may be provided to control the ultrasound imaging device 41 carried on the distal end of the elongated member 65, and the robot arm may be used to control the deliver the radiation therapy 62, the elongate member 65, or the ventilator (not shown). In some circumstances, the patient may be placed on a ventilator (not shown), which may be operated in collaboration with the robot 66 used to move the esophagus 21, since intrathoracic pressure influences the ability to move the esophagus 21.

Similar to other embodiments herein, the elongated member 65 may also include an ultrasound transducer 41 carried on the distal end. The images from the ultrasound transducer 41 may be used for image registration with other imaging modalities such as MRI or CT images by a controller (not shown), also similar to other embodiments herein. The images from the ultrasound transducer 41 may be used to map and track tissue for ablation, both from within the esophagus 21 or externally.

It will be appreciated that elements or components shown with any embodiment herein are exemplary for the specific embodiment and may be used on or in combination with other embodiments disclosed herein.

While the invention is susceptible to various modifications, and alternative forms, specific examples thereof have been shown in the drawings and are herein described in detail. It should be understood, however, that the invention is not to be limited to the particular forms or methods disclosed, but is defined by the appended claims.

## Claims

1. An ablation system to treat cardiac dysrhythmias of a heart within a patient's body (91), comprising:
an elongate member (65) having a proximal end and a distal end (65a) sized to be placed within an esophagus (21) within the patient's body, the elongate member comprising an energy-delivering portion (42) on the distal end;
a machine that delivers external radiation therapy from outside the patient's body to ablate tissue in order to treat the cardiac dysrhythmias, and
a controller coupled to the machine and the energy-delivering portion such that the two energies are delivered during the same procedure to treat cardiac dysrhythmias,
wherein the energy-delivering portion is configured to deliver electroporation energy through the esophagus to ablate tissue in order to treat cardiac dysrhythmias, and
wherein by combining the electroporation energy with the external radiotherapy device, a complete ablation procedure may be performed without substantial risk of injury to critical tissues, including the esophagus, coronary arteries, nerve tissue, and valvular tissue.

2. The system of claim 1, wherein the energy delivery portion comprises electrodes (42) coupled to an electrical pulse generator via the controller to deliver ablation energy by combining ablation from the esophagus with the machine to perform an ablation procedure without substantial risk of injury to critical tissues.

3. The system of any preceding claim, wherein the energy-delivering portion is configured to deliver energy to ablate one or more of cardiac tissue, pulmonary vein tissue, and ganglionic plexi.

4. The system of claim 1, wherein the energy-delivering portion comprises:
at least one electroporation electrode (42); and
an electrical pulse generator electrically coupled to the at least one electroporation electrode.

5. The system of any preceding claim, wherein the elongate member comprises an ultrasound imaging element (41) carried by the distal end (65a), the controller comprising a control processor configured to receive signals from the ultrasound imaging element to generate images to track tissue for ablation.

6. The system of claim 5, wherein the processor is configured to integrate the images from the ultrasound imaging element with magnetic resonance imaging (MRI) or computed tomography (CT) images for image registration to map and track tissue.

7. The system of claim 6, further comprising a ventilator (70), wherein the ventilator is configured to control the patient's respirations to optimize the ablation procedure.

8. The system of claim 7, further comprising at least one robot (66), wherein the at least one robot is configured to position or control one or more of the ultrasound imaging device, the machine for delivering radiation therapy, the distal end of the elongate member, and the ventilator.

9. The system of claim 8, wherein the robot is configured to manipulate the distal end of the elongate member to move the esophagus relative to the heart.

10. The system of any preceding claim, wherein the elongated member (65) includes a motorized control portion (66) on the distal end, capable of moving the esophagus in space and/or rotating the esophagus when actuated.

## Patentansprüche

1. Ablationssystem zur Behandlung von Herzrhythmusstörungen eines Herzes in einem Patientenkörper (91), umfassend:
ein längliches Bauteil (65) mit einem proximalen Ende und einem distalen Ende (65a), das dimensioniert ist, in einer Speiseröhre (21) im Patientenkörper platziert zu werden, wobei das längliche Bauteil einen Energielieferungsabschnitt (42) auf dem distalen Ende umfasst;
eine Maschine, die eine externe Strahlentherapie von außerhalb des Patientenkörpers zum Abtragen von Gewebe bereitstellt, um die Herzrhythmusstörungen zu behandeln, und
einen Controller, der mit der Maschine und dem Energielieferungsabschnitt verbunden ist, so dass die beiden Energien während desselben Eingriffs geliefert werden, um Herzrhythmusstörungen zu behandeln,
wobei der Energielieferungsabschnitt ausgestaltet ist, Elektroporationsenergie durch die Speiseröhre zum Abtragen von Gewebe zu liefern, um Herzrhythmusstörungen zu behandeln, und
wobei durch die Kombination der Elektroporationsenergie mit dem externen Strahlentherapiegerät ein vollständiger Ablationseingriff ohne erhebliches Verletzungsrisiko für kritische Gewebe durchgeführt werden kann, welche die Speiseröhre, Herzkranzgefäße, Nervengewebe und Klappengewebe umfassen.

2. System nach Anspruch 1, wobei der Energielieferungsabschnitt Elektroden (42) umfasst, die mit einem elektrischen Impulsgenerator über den Controller verbunden sind, um Ablationsenergie zu liefern, indem eine Ablation von der Speiseröhre mit der Maschine kombiniert wird, um einen Ablationseingriff ohne erhebliches Verletzungsrisiko für kritische Gewebe durchzuführen.

3. System nach einem der vorhergehenden Ansprüche, wobei der Energielieferungsabschnitt ausgestaltet ist, Energie zum Abtragen von Herzgewebe und/oder Lungenvenengewebe und/oder ganglionäre Plexi zu liefern.

4. System nach Anspruch 1, wobei der Energielieferungsabschnitt umfasst:
mindestens eine Elektroporationselektrode (42); und
einen elektrischen Impulsgenerator, der mit der mindestens einen Elektroporationselektrode elektrisch verbunden ist.

5. System nach einem der vorhergehenden Ansprüche, wobei das längliche Bauteil ein Ultraschall-Bildgebungselement (41) umfasst, das von dem distalen Ende (65a) getragen wird, wobei der Controller einen Steuerprozessor umfasst, der ausgestaltet ist, Signale von dem Ultraschall-Bildgebungselement zu empfangen, um Bilder zum Nachverfolgen von Gewebe für eine Ablation zu erzeugen.

6. System nach Anspruch 5, wobei der Prozessor ausgestaltet ist, die Bilder von dem Ultraschall-Bildgebungselement auf Kernspintomographie (MRI)- oder Computertomographie (CT)-Bilder zur Bilderfassung abzustimmen, um Gewebe abzubilden und nachzuverfolgen.

7. System nach Anspruch 6, das weiter einen Ventilator (70) umfasst, wobei der Ventilator ausgestaltet ist, die Atmungen des Patienten zu steuern, um den Ablationseingriff zu optimieren.

8. System nach Anspruch 7, das weiter mindestens einen Roboter (66) umfasst, wobei der mindestens eine Roboter ausgestaltet ist, das Ultraschall-Bildgebungsgerät und/oder die Maschine zum Bereitstellen einer Strahlentherapie, und/oder das distale Ende des länglichen Bauteils und/oder den Ventilator zu positionieren oder zu steuern.

9. System nach Anspruch 8, wobei der Roboter ausgestaltet ist, das distale Ende des länglichen Bauteils zu manipulieren, um die Speiseröhre relativ zu dem Herz zu bewegen.

10. System nach einem der vorhergehenden Ansprüche, wobei das längliche Bauteil (65) einen motorisierten Steuerabschnitt (66) auf dem distalen Ende umfasst, der in der Lage ist, bei Betätigung die Speiseröhre im Raum zu bewegen und/oder die Speiseröhre zu drehen.

## Revendications

1. Système d'ablation pour traiter les dysrythmies cardiaques d'un cœur à l'intérieur du corps d'un patient (91), comportant :
un organe allongé (65) présentant une extrémité proximale et une extrémité distale (65a) dimensionné pour être placé à l'intérieur d'un œsophage (21) à l'intérieur du corps du patient, l'organe allongé comportant une partie de délivrance d'énergie (42) sur l'extrémité distale ;
une machine qui délivre une radiothérapie externe depuis l'extérieur du corps du patient pour procéder à l'ablation du tissu afin de traiter les dysrythmies cardiaques, et
un dispositif de commande couplé à la machine et à la partie de délivrance d'énergie de sorte que les deux énergies soient délivrées pendant la même procédure pour traiter les dysrythmies cardiaques,
dans lequel la partie de délivrance d'énergie est configurée pour délivrer de l'énergie d'électroporation à travers l'œsophage pour procéder à l'ablation du tissu afin de traiter les dysrythmies cardiaques, et
dans lequel, en combinant l'énergie d'électroporation avec le dispositif de radiothérapie externe, une procédure d'ablation complète peut être réalisée sans risque substantiel de lésion des tissus critiques, y compris l'œsophage, les artères coronaires, le tissu nerveux et le tissu valvulaire.

2. Système selon la revendication 1, dans lequel la partie de délivrance d'énergie comporte des électrodes (42) couplées à un générateur d'impulsions électriques via le dispositif de commande pour délivrer de l'énergie d'ablation en combinant l'ablation depuis l'œsophage avec la machine pour réaliser une procédure d'ablation sans risque substantiel de lésion des tissus critiques.

3. Système selon une quelconque revendication précédente, dans lequel la partie de délivrance d'énergie est configurée pour délivrer de l'énergie pour procéder à l'ablation d'un ou plusieurs parmi le tissu cardiaque, le tissu veineux pulmonaire et les plexi ganglionnaires.

4. Système selon la revendication 1, dans lequel la partie de délivrance d'énergie comporte :
au moins une électrode d'électroporation (42) ; et
un générateur d'impulsions électriques couplé électriquement à l'au moins une électrode d'électroporation.

5. Système selon une quelconque revendication précédente, dans lequel l'organe allongé comporte un élément d'imagerie par ultrasons (41) porté par l'extrémité distale (65a), le dispositif de commande comportant un processeur de commande configuré pour recevoir des signaux de l'élément d'imagerie par ultrasons pour générer des images pour suivre le tissu à des fins d'ablation.

6. Système selon la revendication 5, dans lequel le processeur est configuré pour intégrer les images provenant de l'élément d'imagerie par ultrasons avec des images d'imagerie par résonance magnétique (IRM) ou de tomodensitométrie (TDM) pour l'enregistrement d'image pour cartographier et suivre le tissu.

7. Système selon la revendication 6, comportant en outre un ventilateur (70), dans lequel le ventilateur est configuré pour contrôler les respirations du patient pour optimiser la procédure d'ablation.

8. Système selon la revendication 7, comportant en outre au moins un robot (66), dans lequel l'au moins un robot est configuré pour positionner ou commander un ou plusieurs parmi le dispositif d'imagerie par ultrasons, la machine pour administrer la radiothérapie, l'extrémité distale de l'organe allongé et le ventilateur.

9. Système selon la revendication 8, dans lequel le robot est configuré pour manipuler l'extrémité distale de l'organe allongé pour déplacer l'œsophage par rapport au cœur.

10. Système selon une quelconque revendication précédente, dans lequel l'organe allongé (65) comprend une partie de commande motorisée (66) sur l'extrémité distale, capable de déplacer l'œsophage dans l'espace et/ou de faire tourner l'œsophage lorsqu'il est actionné.
